# EUROPEAN PATENT APPLICATION

(11) **EP 1 656 993 A1**
(43) Date of publication of application: **17.05.2006**
(21) Application number: 04078024.9
(22) Date of filing: 03.11.2004
(51) Int. Cl.: B01J 37/08, B01J 37/30, B01J 29/068, B01J 29/12, C07C 2/58

(54) **Alkylation catalyst, its preparation and use**

(71) Applicant: Albemarle Netherlands B.V., 3818 LE Amersfoort (NL)
(72) Inventor: Van Broekhoven, Emanuel Hermanus, 1141 DN Monnickendam (NL); Steenwinkel, Edgar Evert, 2011 WD Haarlem (NL); Boomert, Arjan Peter, 1822 KE Alkmaar (NL); Harte, Mark Hendrikus, 1507 TB Zaandam (NL); Alofs-Gimpel, Danielle, 1531 SP Wormer (NL)
(74) Representative: Rasser, Jacobus Cornelis

(57) **Abstract**

Process for the preparation of a catalyst comprising the steps of: (a) calcining solid acid-containing particles at a temperature in the range of 400-575°C, (b) incorporating a Group VIII noble metal into the calcined particles to form noble metal-containing particles, and (c) calcining the noble metal-containing particles at a temperature in the range of 350-600°C.

The use of two calcination steps in the above temperature ranges results in alkylation catalysts with improved performance.

## Description

The present invention relates to a process for the preparation of a catalyst suitable for alkylating a hydrocarbon feed. The invention further relates to the catalyst so obtained, and its use in alkylation processes.

Within the framework of the present invention, the term alkylation refers to the reaction of an alkylatable compound, such as an aromatic or saturated hydrocarbon, with an alkylation agent, such as an olefin. Without limiting the scope of the invention, we will further illustrate the invention by discussing the alkylation of saturated hydrocarbons, in general branched saturated hydrocarbons, with an olefin to give highly branched saturated hydrocarbons with a higher molecular weight. Hydrocarbons contain no atoms other than hydrogen and carbon.
This reaction is of interest because it makes it possible to obtain, through the alkylation of isobutane with an olefin containing 2-6 carbon atoms, an alkylate which has a high octane number and which boils in the gasoline range. Unlike gasoline obtained by cracking heavier petroleum fractions such as vacuum gas oil and atmospheric residue, gasoline obtained by alkylation is essentially free of contaminants such as sulfur and nitrogen and so has clean burning characteristics. Its high anti-knock properties, represented by the high octane number, lessen the need to add environmentally harmful anti-knock compounds such as aromatics or lead. Also, unlike gasoline obtained by reforming naphtha or by cracking heavier petroleum fractions, alkylate contains few if any aromatics or olefins, which, environmentally speaking, is a further advantage.

The alkylation reaction is acid-catalysed. At present, in commercial alkylation equipment use is made of liquid acid catalysts such as sulfuric acid and hydrogen fluoride. The use of such catalysts is attended with a wide range of problems. For instance, sulfuric acid and hydrogen fluoride are highly corrosive, so that the equipment used has to meet high quality requirements. Since the presence of highly corrosive materials in the resulting fuel is objectionable, the remaining acid has to be removed from the alkylate. Also, because of the phase separations which have to be carried out, the process is complicated and thus expensive. Besides, there is always the risk that toxic substances such as hydrogen fluoride will be emitted.

A more recent development in this field is the use of solid acid catalysts, such as zeolite-containing catalysts. WO 98/23560 discloses the use in the alkylation of hydrocarbons of a catalyst containing a zeolite, such as a Y zeolite, a Group VIII noble metal (e.g., platinum or palladium) as hydrogenation component, and optionally a matrix material, such as alumina.
Such a catalyst can be prepared by mixing the solid acid with matrix material, shaping the mixture to form particles, and calcining the particles. The hydrogenation component may be incorporated into the catalyst composition by impregnation of said particles.

EP 1 308 207 discloses an alkylation process using a catalyst comprising a solid acid, a hydrogenation component consisting essentially of one or more Group VIII noble metals, and at least 0.05 wt% of sulfur. This catalyst is prepared by contacting a material comprising the solid acid and the hydrogenation component with a sulfur-containing compound.
This document discloses different methods for preparing the material comprising the solid acid and the hydrogenation component, one of these methods involving the steps of:
(i) shaping, e.g. extruding, the solid acid, optionally after mixing it with a matrix material, to form particles,
(ii) calcining the resulting particles, and
(iii) incorporating the hydrogenation component into the calcined particles by, e.g., impregnating the particles with a solution of one or more Group VIII noble metals and/or by (competitive) ion exchange.

The so-prepared material is preferably calcined and reduced prior to its contact with the sulfur-containing compound.

It has now been found that the performance in alkylation reactions of noble metal-containing solid acid catalysts can be further improved if the calcination steps before and after incorporation of the hydrogenation component - i.e. steps a) and c) - are both conducted in a specific temperature window.

The present invention therefore relates to a process for the preparation of a catalyst comprising the steps of:
a. calcining solid acid-containing particles at a temperature in the range of 400-575°C,
b. incorporating a Group VIII noble metal into the calcined particles to form noble metal-containing particles, and
c. calcining the noble metal-containing particles at a temperature in the range of 350-600°C.

As illustrated by the Examples below, it is important that the temperature during both the first and the second calcination step is in the claimed temperature window.

### The solid acid-containing particles

The solid acid-containing particles generally comprise a solid acid and a matrix material.
Examples of suitable solid acids are zeolites such as zeolite beta, MCM-22, MCM-36, mordenite, X-zeolites and Y-zeolites, including H-Y-zeolites and USY-zeolites, non-zeolitic solid acids such as silica-alumina, sulfated oxides such as sulfated oxides of zirconium, titanium, or tin, mixed oxides of zirconium, molybdenum, tungsten, phosphorus, etc., and chlorinated aluminium oxides or clays. Preferred solid acids are zeolites, including mordenite, zeolite beta, X-zeolites and Y-zeolites, the latter including H-Y-zeolites and USY-zeolites. Mixtures of solid acids can also-be employed. An even more preferred solid acid is Y-zeolite with a unit cell size of 24.34 - 24.72 angstroms, and most preferred is a Y-zeolite with a unit cell size of 24.42-24.56 angstroms.
Examples of suitable matrix materials are alumina, silica, titania, zirconia, clays, and mixtures thereof. Matrix materials comprising alumina are generally preferred.

Preferably, the solid acid-containing particles comprise 2-98 wt% of the solid acid and 98-2 wt% of the matrix material, based on the total weight of the solid acid and the matrix material present in the particles. More preferably, the solid acid-containing particles comprise 10-90 wt% of the solid acid and 90-10 wt% of the matrix material. Even more preferably, the solid acid-containing particles comprise 10-80 wt% of the matrix material and balance solid acid, most preferably they comprise 10-40 wt% of the matrix material and balance solid acid, based on the total weight of the solid acid and the matrix material contained in the particles.

The solid acid-containing particles can be prepared by standard methods, e.g. mixing a solid acid and a matrix material and shaping the mixture to form shaped bodies. A preferred shaping method is extrusion, but also agglomeration, spray drying, and beads formation by, e.g., the oil droplet method can be used.
Suitable shapes of said particles include spheres, cylinders, rings, and symmetric or asymmetric polylobes, for instance tri- and quadrulobes. Preferably, the catalyst particles have an average particle diameter of at least 0.5 mm, more preferably of at least 0.8 mm, and most preferably of at least 1.0 mm. The upper limit of the average particle diameter preferably lies at 10.0 mm, more preferably at 5.0 mm, even more preferably at 3.0 mm.

### Step a)

The solid acid-containing particles are calcined at a temperature in the range of 400-575°C, preferably 450-550°C, more preferably 460-500°C.
The heating rate preferably ranges from 0.1 to 100°C/min, more preferably 0.5°C to 50°C/min, most preferably 1 to 30°C/min.
Calcination is preferably conducted for 0.01-10 hrs, more preferably 0.1-5 hrs., most preferably 0.5-2hrs
It is preferably conducted in an air and/or inert gas (e.g. nitrogen) flow. More preferably, this atmosphere is dry.

Preferably, the solid acid-containing particles are dried before being calcined. This drying is preferably conducted at a temperature of about 110-150°C.

The calcination can be performed in any equipment, such as a fixed bed reactor, a fluidised bed calciner, and a rotating tube calciner.

### Step b)

A Group VIII noble metal is then incorporated into the calcined solid acid-containing particles. This is preferably done by impregnation or competitive ion exchange of the solid acid-containing particles using a solution comprising Group VIII noble metal ions and/or their complexes and (optionally) NH4+ ions. Preferred Group VIII noble metals are platinum, palladium, and combinations thereof. More preferably, at least one of the Group VIII noble metals is platinum.
Suitable Group VIII noble metal salts include nitrates, chlorides, and ammonium nitrates of the noble metals or their complexes (e.g. NH₃ complexes).

### Step c)

The resulting noble metal-containing particles are then calcined at a temperature in the range of 350-600°C, preferably 400-550°C, most preferably 450-500°C.

This temperature is preferably reached by heating the particles by 0.1-100°C/min, more preferably 0.5-50°C/min, most preferably 1-30°C/min to the desired final value between 350 and 600°C.
Calcination is preferably conducted for 0.01-10 hrs, more preferably 0.1-5 hrs, most preferably 0.5-2 hrs.
Calcination is preferably conducted in an air and/or inert gas (e.g. nitrogen) flow. More preferably, this atmosphere is dry.

Optionally, a separate drying step is applied between steps (b) and (c). Alternatively, the noble metal-containing particles are dried during the calcination step.
Also optionally, a dwell of about 15-120 minutes, preferably 30-60 minutes is introduced at a temperature of about 200-250°C

After calcination step (c), the resulting catalyst particles are preferably reduced at a preferred temperature range of 200 to 500°C, more preferably 250 to 350°C, in a reducing gas such as hydrogen.
Before or after this reduction treatment, water may be added to the catalyst particles. As described in non-prepublished European Patent Application No. 04075387.3, the presence of 1.5-6 wt% of water, more preferably 1.8-4, and most preferably 2-3 wt% - measured as the loss on ignition at 600°C - has a positive effect on the alkylation activity and the alkylate quality.

### The alkylation process

Preferably, the hydrocarbon to be alkylated in the alkylation process is a branched saturated hydrocarbon such as an isoalkane having 4-10 carbon atoms. Examples are isobutane, isopentane, isohexane or mixtures thereof, with isobutane being most preferred. The alkylation agent preferably is an olefin having 2-10 carbon atoms, more preferably 2-6 carbon atoms, still more preferably 3-5 carbon atoms, and most preferably 4 carbon atoms. Most preferably, the alkylation process consists of the alkylation of isobutane with butenes.

As will be evident to the skilled person, the alkylation process can take any suitable form, including fluidised bed processes, slurry processes, and fixed bed processes. The process can be carried out in a number of beds and/or reactors, each with separate addition of alkylation agent if desirable. In such a case, the process of the
invention can be carried out in each separate bed or reactor.

Suitable process conditions are known to the skilled person. Preferably, an alkylation process as disclosed in WO 98/23560 is applied. In this process the catalyst is subjected intermittently to a mild regeneration step by being contacted with a feed containing a saturated hydrocarbon and hydrogen. This mild regeneration is preferably carried out at 90% or less of the active cycle of the catalyst, whereby the active cycle is defined as the time from the start of the feeding of the alkylation agent to the moment when, in comparison with the entrance of the catalyst-containing reactor section, 20% of the alkylation agent leaves the catalyst-containing reactor section without being converted, not counting
isomerisation inside the molecule.

The process conditions applied in the present process are summarised in the following Table:

| | Temperature range of [°C] | Pressure range of [bar] | Molar ratio of hydrocarbon to alkylation agent |
|---|---|---|---|
| Preferred | -40 - 250 | 1-100 | 5:1 - 5,000:1 |
| More preferred | 20 - 150 | 5 - 40 | 50:1 - 1,000:1 |
| Most preferred | 65 - 95 | 15 - 30 | 150:1 - 750:1 |

This mild regeneration is preferably conducted at temperatures and pressures that differ from the reaction temperature by not more than 50%, more preferably by not more than 20%, still more preferably by not more than 10%.

Optionally, in the above process the catalyst particles may be subjected to a high-temperature regeneration with hydrogen in the gas phase. This high-temperature regeneration is preferably carried out at a temperature of at least 150°C, more preferably at 150° - 600°C, and most preferably at 200° - 400°C. For details of this regeneration procedure, reference is made to WO 98/23560. The high-temperature regeneration can be applied periodically during the alkylation process and is preferably applied after every 50, more preferably after every 100, and most preferably after every 200-400 mild regenerations.
If as a result of high-temperature regeneration the water content of the catalyst particles has decreased to below the desired level, the catalyst particles may be rehydrated during the process in the ways described above.

Preferably, in addition to the high-temperature regeneration treatment a milder regeneration is applied during the alkylation process, such as described in WO 98/23560, in particular page 9, line 13 through page 13, line 2. This text passage is incorporated herein by reference. More in particular, during the alkylation process the catalyst particles are preferably subjected intermittently to a regeneration step by being contacted with a feed containing a hydrocarbon and hydrogen, with said regeneration preferably being carried out at 90% or less, more preferably at 60% or less, even more preferably at 20% or less, and most preferably at 10% or less of the active cycle of the catalyst. The active cycle of the catalyst is defined as the time from the start of the feeding of the alkylation agent to the moment when, in comparison with the alkylation agent added to the catalyst-containing reactor section, 20% of the alkylation agent leaves the catalyst-containing reactor section without being converted, not counting isomerisation inside the molecule.

The quality of the alkylate product obtained in the process according to the invention can be measured by its Research Octane Number (RON). The RON is a measure of the anti-knock rating of gasoline and/or gasoline constituents. The higher the RON, the more favourable the anti-knock rating of the gasoline will be. Depending on the type of gasoline engine, generally speaking a higher anti-knock rating is of advantage when it comes to the working of the engine. The product obtained in the process according to the invention preferably has a RON of 90 or higher, more preferably of 92 or higher, most preferably 94 or higher. The RON is obtained by determining, e.g. via gas chromatography, the percentages by volume of the various hydrocarbons in the product. The percentages by volume are then multiplied by the RON contribution and added up.
Examples of compounds with a RON of 90 or higher are isopentane, 2,2-dimethyl butane, 2,3-dimethyl butane, trimethyl butane, 2,3-dimethyl pentane, 2,2,4-trimethyl pentane, 2,2,3-trimethyl pentane, 2,3,4-trimethyl pentane, 2,3,3-trimethyl pentane, and 2,2,5-trimethyl hexane.

### EXAMPLES

### Examples 1-6

Dried extrudates comprising 70 wt% of USY-zeolite and 30 wt% of an alumina matrix were calcined in air at different final temperatures for about 1 hour after being heated at a rate of about 5°C/min. The calcination temperatures applied are listed in Table 1 as "T calcination 1".
The :calcined extrudates were subsequently impregnated with an aqueous solution of Pt(NH₃)₄Cl₂ and NH₄NO₃ by incipient wetness. The amount of NH₄+ ions was equivalent to the number of Na⁺-exchangeable sites of the catalyst.
After drying at 120°C for 2 hours the impregnated extrudates were calcined for 2 hours in air at different final temperatures ("T calcination 2" in Table 1) to obtain catalyst particles. The heating rate to the final temperature was about 5°C/min, (with a dwell of about 2 hrs at 230°C).
The resulting Pt-content of the extrudates was 0.34 wt%.

These catalyst particles were subsequently tested according to the following procedure.
A fixed-bed recycle reactor as described in WO 98/23560 having a diameter of 2 cm was filled with a 1:1 volume/volume mixture of 38.6 grams of catalyst extrudates (wetted in ambient air to a Loss On Ignition (600°C) of about 4.5 wt%) and carborundum particles (60 mesh). At the centre of the reactor tube a thermocouple of 6 mm in diameter was arranged. The reactor was flushed with nitrogen for 30 minutes (21 NI/hour). Next, the system was tested for leakages at elevated pressure, after which the pressure was raised to 21 bar and the nitrogen replaced by hydrogen (21 NI/hour). The reactor temperature was then raised to 275°C at a rate of 1°C/min and the catalyst was reduced at 275°C. After 2 hours, the reactor temperature was lowered to the reaction temperature.
The hydrogen stream was stopped with the attaining of the reaction temperature. Isobutane was supplied to the reactor at a rate of about 4,000 grams/hour. About 95 - 98% of the isobutane was fed back to the reactor. About 2 - 5% was drained off for analysis. Such an amount of isobutane was supplied to the reactor as to ensure a constant quantity of liquid in the system. When the system had stabilised, such an amount of cis-2-butene was added to it as to give a cis-2-butene-WHSV of 0.19 (calculated on zeolite weight in the catalyst sample). The overall rate of flow of liquid in the system was maintained at about 4,000 g/h. The weight ratio of isobutane to cis-2-butene at the reactor inlet was about 750. The pressure in the reactor amounted to 21 bar.
Each time after 1 hour of reaction, the catalyst particles were regenerated by being washed with isobutane for 5 minutes, followed by 50 minutes of regeneration through being contacted with a solution of 1 mole% of H₂ in isobutane, and then being washed with isobutane for another 5 minutes (total washing and regeneration time 1 hour). After this washing step, alkylation was started again. The temperature during the washing steps, the regeneration step, and the reaction step was the same.
After processing as above for 24 hours at the same temperature, a pseudo-steady state was reached. Then, the temperature was decreased and the process was conducted as above for another 24 hours. Hence, the catalytic performance was measured at various temperatures going from higher to lower.

The performance was characterised by the reaction temperature and the research octane number (RON) at 99.5% olefin conversion per reactor pass. The RON was determined as described on pages 13 and 14 of WO 9823560, the only exception being that the RON contribution of total C9+ (excl. 2,2,5-trimethylhexane) was estimated to be 84 instead of 90. The C5+ alkylate yield is defined as the weight amount of C5+ alkylate produced divided by the overall weight of olefin consumed.

The effect of the calcination temperatures on the performance of the catalyst particles is indicated in Table 1:

**Table 1**

| Example | T calcination 1 (°C) | T calcination 2 (°C) | Reaction T at 99.5% conv. (°C) | RON at 99.5% conv. |
|---|---|---|---|---|
| 1 | 450 | 450 | 61 | 96.7 |
| 2 | 475 | 450 | 52 | 97.7 |
| 3 | 500 | 450 | 57 | 97.3 |
| 4 | 540 | 450 | 58 | 97.2 |
| 5 | 500 | 600 | 60 | 97.0 |
| 6 | 600 | 450 | 64 | 96.6 |

Table 1 clearly shows that the performance of the alkylation catalyst can be optimised by varying the calcination temperature both before and after impregnation. Application of calcination temperatures in the claimed range results in improved alkylation catalysts.

## Claims

1. Process for the preparation of a catalyst which is suitable for alkylating a hydrocarbon feed, which process comprises the steps of:
a. calcining solid acid-containing particles at a temperature in the range of 400-575°C,
b. incorporating a Group VIII noble metal into the calcined particles to form noble metal-containing particles, and
c. calcining the noble metal-containing particles at a temperature in the range of 350-600°C.

2. A process according to claim 1 wherein the temperature applied in step a) is in the range of 450-550°C.

3. A process according to claim 2 wherein the temperature is in the range of 460-500°C.

4. A process according to any one of the preceding claims wherein the temperature applied in step c) is in the range of 400-550°C.

5. A process according to claim 4 wherein the temperature is in the range of 450-500°C.

6. A process according to any one of the preceding claims wherein the solid acid is a zeolite selected from the group consisting of mordenite, zeolite beta, X-zeolites, and Y-zeolites.

7. Catalyst obtainable by the process of any one of the preceding claims.

8. Use of the catalyst of claim 7 for the alkylation of hydrocarbons.

9. Use according to claim 8 wherein the hydrocarbons are saturated hydrocarbons.
